# EUROPEAN PATENT SPECIFICATION

(11) **EP 0 002 004 B1**
(45) Date of publication and mention of the grant of the patent: **24.03.1982**
(21) Application number: 78101284.4
(22) Date of filing: 02.11.1978
(51) Int. Cl.: C07C 149/24, C07D 213/20, C07D 295/08, C11D 1/62

(54) **Process for the manufacture of fluorinated cationic compounds and their use as surfactants**
Verfahren zur Herstellung fluorhaltiger kationischer Verbindungen und ihre Verwendung als oberflächenaktive Mittel
Procédé pour la préparation de dérivés cationiques fluorés et leur utilisation comme composés tensio-actifs

(30) Priority: 07.11.1977 US 849205
(43) Date of publication of application: 30.05.1979
(73) Proprietor: CIBA-GEIGY AG, 4002 Basel (CH)
(72) Inventor: Cooke, Thomas W., Mahopac New York 10541 (US); Kleiner, Eduard K., New York N.Y. 10021 (US); Dear, Robert Ernest Arthur, Mount Kisco New York 10549 (US)

## Description

The present invention relates a process for the manufacture of fluorinated cationic compounds, and their use as surfactants.

U.S. patent specifications 3,883,596, 3,893,984, 3,899,484, 3,906,049, 3,948,887, 3,976,698, and German Offenlegungsschriften 2,018,461 and 2,342,888 show the preparation of possible precursors (tertiary amino group containing compounds) to the fluorinated cationic surfactants described in this specification. However, they are all prepared by an entirely different, cumbersome, and less efficient synthesis route.

Synthesis route shown in the above references: 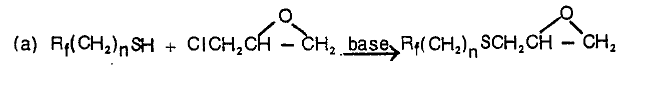 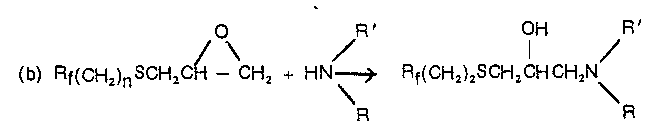 wherein the cited synthesis route requires two steps (plus a third step to obtain a surfactant) and requires the formation of fluorinated chlorohydrin or epoxide intermediates, the novel compounds according to the invention are obtained by only one reaction step from the R_{f-}thiol to the finished cationic surfactant with no isolatable intermediates. The reactions of this invention can also be done in solution in quantitative yields with no work-up operations while the prior art process yields are lower.

It is one object of the present invention to provide a process for the manufacture of new fluorinated cationic compounds .of the formula 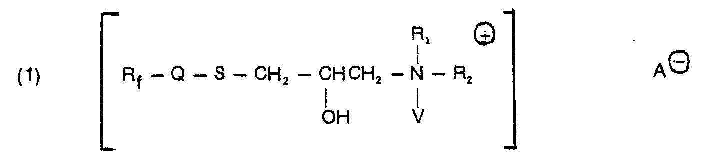 wherein R_{f} is perfluoroalkyl of 1 to 18 carbon atoms or said perfluoroalkyl substituted by perfluoroalkoxy of 2 to 6 carbon atoms, Q is alkylene of 1 to 12 carbon atoms, alkylene thioalkylene of 2 to 12 carbon atoms, alkylene oxyalkylene of 2 to 12 carbon atoms, or alkylene iminoalkylene of 2 to 12 carbon atoms, wherein the imino group is optionally substituted by alkyl of 1 to 6 carbon atoms, V, R, and R₂ indepedently of one another represent alkyl of 1 to 20 carbon atoms which is optionally substituted by hydroxyl, cyano, halogen, or by alkoxy of 1 to 4 carbon atoms, or cycloalkyl, benzyl, phenyl or polyalkyleneoxy of 2 to 20 alkoxy units and alkylene units of 2 to 4 carbon atoms, or R, and R₂ together with the nitrogen atom to which they are attached, represent a 5- or 6-membered heterocyclic radical or R,, R₂ and V, together with the nitrogen atom which links them represent a pyridine ring and A^{Θ} represents the anion of an organic or inorganic acid, which comprises reacting a mercaptan of the formula (2) R_{f}―Q―SH with a compound of the formula 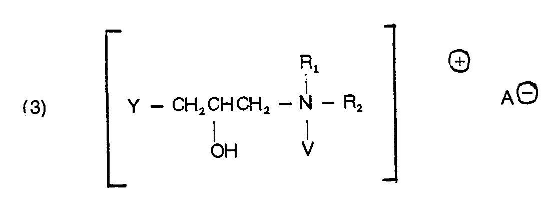 wherein R_{f} Q, R,, R₂, V and A⁹ have the meanings indicated above and Y represents halogen.

Another object of the invention is the use of compounds of formula (1) as surfactants.

In formula (1), Rf represents preferably a perfluoroalkyl group of 3 to 18, preferably 4 to 14 carbon atoms, wherein the alkyl unit may be a straight or branched chain alkyl group. R_{f} may also be a mixture of said perfluoroalkyl groups. Examples of perfluoroalkyl groups R_{f} are perfluoropropyl, perfluorobutyl, perfluoropentyl, perfluorohexyl, perfluorooctyl, perfluorodecyl, perfluorododecyl, perfluorohexadecyl of perfluorooctadecyl.

Q represents preferably alkylene or alkylsubstituted alkylene with 2 to 8 carbon atoms, most preferably C₂―C₃-alkylene and especially ethylene. The alkyl substituents may be lower alkyl of 1 to 4. carbon atoms.

The radicals R₁, R₂ and V can be different from each other or they are preferably identical. When radicals R₁, r₂ and V represent alkyl, they may be straight or branched alkyl groups. Examples of said alkyl groups are methyl, ethyl, n-propyl, isopropyl, n-butyl, sec-butyl, hexyl, octyl, dodecyl or. octadecenyl. Substituted alkyl groups V, R, and R₂ are in particular haloalkyl, cyanoalkyl, hydroxyalkyl or alkoxyalkyl, each preferably containing 2 to 4 carbon atoms, for example 2-chloroethyl, 2-cyanoethyl, 2-hydroxyethyl, 3-hydroxypropyl, β-methoxyethyl or β-ethoxypropyl.

Examples of cycloalkyl in the meaning of R,, R₂ and V are cyclopentyl or preferably, cyclohexyl.

In a preferred embodiment, the radicals R₁, R₂ and V are alkyl groups of 1 to 4 carbon atoms and most preferably methyl or ethyl groups.

If the substituents R, and R₂ together with the common nitrogen atom, represent a heterocyclic radical, this is for example, pyrrolidino, piperidino, pipecolino, morpholino, thiomorpholino or piperazino.

Possible anions AS are both anions of inorganic acids for example, the chloride, bromide, fluoride, iodide, sulphate or phosphate ion and of organic acids, for example or aromatic or aliphatic sulphonic acids such as the benzene sulphonate, p-toluenesulphonate, methanesulphonate or ethane- sulphonate ion, and also the anions or acid alkyl ester of inorganic acids, such as the methosulphate or ethosulphate ions, or carboxylic acids, e.g. acetate, propionate, or benzoate ions.

The anion A^{e} preferably denotes chloride, bromide, iodide methylsulphate or ethylsulphate ion.

Y represents halogen such as chlorine, bromine, fluorine or iodine.

The inventive process is preferably used for preparing fluorinated cationic compounds of the formula 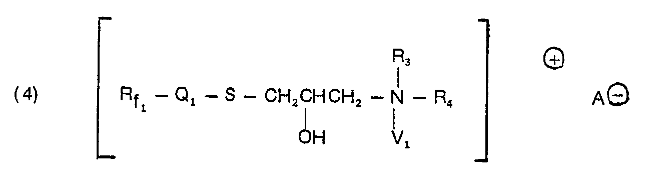 wherein R_{f}, represents a perfluoroalkyl group of 1 to 18 carbon atoms, Qₗ is alkylene or 2 or 3 carbon atoms, each of R₃ and R₄ independently represents alkyl of 1 to 4 carbon atoms, and Vₜ represent alkyl of 1 to 4 carbon atoms or benzyl and, A^{Θ} has the given meaning or particularly interesting fluorinated cationic compounds of the formula 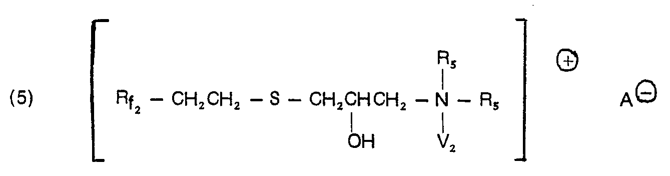 wherein R_{f2} represents a perfluoroalkyl group of 4 to 14 carbon atoms and each of V₂ and Rₛ represents methyl or ethyl and A⁸ has the given meaning.

Amongst these compounds of the formula (5), those in which R_{f2} represents perfluorobutyl, perfluorohexyl, perfluorooctyl, perfluorodecyl, perfluorododecyl, perfluorotetradecyl or a mixture of these substituents, V₂ and R₅ represent methyl of ethyl and A^{Θ} represents a chloride ion are particularly preferred.

The process for the manufacture of compounds of formula (1) is preferably carried out by bringing the reactants to reaction in a mixture of water and a water-soluble cosolvent in the presence of a base. The co-solvent in the presence of a base. The co-solvent is useful to aid the solubilization of the R_{f}―Q―thiol in the aqueous phase. The heterogeneous mixture is then stirred while the base is added into the reaction mixture. A mildly exothermic reaction generally occurs and, if the proper solvent system is selected, a homogeneous solution results containing the fluorinated cationic sufactant and the by-product halide salt of the base.

An example of a starting compound of formula (3) is the commercially available compound, 3-chloro-2-hydroxypropyl-trimethylammonium chloride (generally obtained as a 52% by weight aqueous solution).

The co-solvents for this process are those which are water miscible and in which the R_{f}―Q―thiol is partially or completely soluble. The following are examples of such suitable solvents: methanol, ethanol, 2-propanol, butyl carbitol, ethylene glycol, propylene glycol, hexylene glycol, glycol ethers, ethylene glycol monomethyl or monoethyl ether, ethylene glycol dimethyl ether, diethylene glycol, triethylene glycol, tetrahydrofuran, methyl cellosulve acetate, tetramethylene sulfone, acetone, diacetone alcohol, N-methyl pyrrolidone and the like. Preferable solvents are water soluble sec- and tert-alcohols. The most preferable solvent is hexylene glycol.

The ratio of the water miscible Rₜ₋Q-thiol co-solvent to water is an important factor in this invention, if a homogeneous liquid composition is to be obtained. If an insufficient amount of the solvent is employed, the resulting composition is a gel. If an excess of the solvent is used, a nonhomogeneous product results (2 layers). The specific ratio of the R_{f-}Q-thiol solvent to water also depends on the solubility of the R_{f-}Q-thiol, the solubility of the surfactant end-product and the solubility of the by-product salt. For example, if hexylene glycol is the co-solvent, in a system at 33% solids, the preferable ratio of water to hexylene glycol is from 7.5:1 to 4.5:1.

Since it is clear from the above that the specific ratio of the co-solvent to water depends on many factors, it is impossible to predict the acceptable ratio limits for every solvent. A routine method to determine the acceptable ratios for various solvents is to carry out a series of trial reactions, at a specific solids content, varying the amounts of water and solvent and observing whether the resulting product is homogeneous, gelled or heterogeneous. In general, if the product is a gel, then the amount of the solvent should be increased and the amount of water decreased. If the product is heterogeneous (2 layers), the solvent should be decreased and the amount of water increased.

The base selected for this process should be one that is capable of abstracting the by-product hydrogen halide (HY) and form the resulting halide salt. The base may be either inorganic or organic and result in the formation of soluble or insoluble by-product halide salts. The soluble by-product salts remain in the final surfactant solution while insoluble salts may be removed by filtration. The base may be added into the reaction mixture neat or in solution.

The following is an illustrative list of bases for this reaction: a) for soluble by-product salts - potassium hydroxide, sodium hydroxide, lithium hydroxide, calcium hydroxide, sodium carbonate, sodium bicarbonate, triethylamine, pyridine, trimethylamine, lutidine and N-methyl-diethanolamine; b) for insoluble by-product salts, many basic ion-exchange resins may be used - such as Amberlite^{*} IRA-93 and IRA-94, Stratabed^{*} 93, Amberlyst^{*} A-27 (OH form), Rexyn^{*} 203 and Rexyn^{*} 201 (OH form). These basic ion-exchange resins all contain tertiary amine or quaternary ammonium hydroxide functionally.

The general reaction process is one that is unique and novel in several respects: a) It is very facile. b) It is basically a one-step process. c) It is usually quantitative. d) It does not use any previously claimed fluorochemical intermediate chlorohydrins, epoxides, or amines; and as such, the expensive fluorochemical component (i.e. the R_{f-}Q-thiol) is not subject to the handling and yield losses of intermediate synthesis steps.

In a preferred embodiment of the manufacturing process, the R_{f-}Q-thiol, 3-halo-2-hydroxypropyl-ammonium halide, water and elected co-solvent are placed in the reaction vessel and stirred at an appropriate temperature (room temperature is the preferred). The system is generally heterogeneous (2 layers) at this stage. Then an equivalent amount of base is gradually added with stirring. A mild exotherm usually results. The system becomes homogeneous shortly thereafter indicating the completion of the reaction. The system may also be stirred for an additional length of time at an elevated temperature if necessary. With the proper choice of solids content, co-solvent (type and amount, and base, a clear homogeneous solution results. The resulting product of the formula (1) can be isolated if desired.

The fluorinated cationic compounds of the formula (1), (4) and (5) are valuable surfactants. They demonstrate the properties of excellent water solubility and dramiatic lowering of the surface tension of aqueous solutions, even at very low concentrations, e.g. 20 dynes/cm at 0.1% active substances.

The new surfactants are stable and effective in neutral, acidic and alkaline aqueous solutions.

The novel surfactants of this invention are useful to improve or impart properties such as: wetting, penetration, spreading, leveling, foam stability, flow properties, emulsification, dispersion, and oil and water repellency. Based on these unique properties are numerous applications, some of which follow. Although applications are suggested for a particular use area, the general applicability of each concept is inferred for other applications.

### Plastics and rubber industry

### Emulsifying agent for polymerization, particularly fluoromonom

As a latex stabilizer

To aid in the preparation of agglomerates of powdered fluorocarbon polymers

In synergistic mixtures with hydrocarbon surfactants to wet low energy surfaces including natrual and synthetic rubbers, resins, plastics

As an adjuvant for foam applications and as foaming agents to aid in leak detection

As a foam additive to control spreading, crawling, edge build up
^{*} Reqistered Trademark

As mould release agents, for silicons, etc.

In refractory processes

As an anti-mist film former

Additive for elimination of trapped air in plastic laminates

Wetting agent for resin molds for definition, strength

Hot-melt additive for oil and grease repellency

Resin additive for improved wetting of and bonding with fillers

Flow modifier for extruding hot melts; spreading, uniformity, anticratering

Adjuvant for resin etchant

Mold release agent, demoulding agent

Retarder for plasticizer migration or evaporation

Internal antistatic agent for polyolefins

Antiblocking agent for polyolefins

### Petroleum Industry

Wetting. assistant for oil well treatments, drilling muds

As a film evaporation inhibitor for gasoline, jet fuel, solvents, hydrocarbons

Lubricating, cutting oil improver, to improve penetration times

In extreme pressure lubricants

Oil spill collecting agent

Additive to improve tertiary oil recovery

### Textile and leather Industries

Soil release and soil proofing agent

Oil/water repellent textile and leather treatment

Wetting agent to improve coverage and penetration of pores of substrates

Anti-foaming agent in textile treatment baths

Wetting agent for finish-on-yarn uniformity

Penetrating agent for finishes on tow, heavy denier fibers

Emulsifying agent/lubricant/for fiber finishes

Cleaner/metal treating agent for polymerization equipment

Flow modifier for spinning of hot melts, solutions

Additive for fabric finishes for spreading, uniformity

Wetting agent for dyeing

Penetration aid for bleaches

Wetting agent for binder in nonwoven fabrics

### Paint, pigment and finishing Industries

Leveling, anti-cratering adjuvant for finishes and paint

Adjuvant for control of soiling

Agent to control differential evaporation of solvents

Leveling agent for floor waxes

Adjuvant for waxes to improve oil and water repellency

Adhesion improver for oily or greasy surfaces

To combat pigment flotation problems

Improver for automotive finishes, based on water-based coatings in which the pigments are rendered non-reactive

Pigment grinding aid to promote wetting, dispersion, color development

Foam generator substance for the application of dyes, inks

Electrolytic conversion coatings

### Mining and metalworking Industries

In cleaning agents for property improvement

Additive for solvent cleaning

Additive for metal pickling baths to increase bath life and acid runoff

Additive for chrome electroplating: surface tension reduction, foaming

Additive for soldering flux, especially for electronic circuitry

Protective agent for coatings (tarnish resistance, grease repellency)

Corrosion inhibitor

Additive for etchant solution for improved definition

To form antimist films and anti-condensation surfaces

Plastic preplate and silicon etchant technology

In soldering flux for microelectronics to reduce foaming

In chemical-roughing agent solutions, prior to galvanisation

As a colloidal dispersion aid for magnetic solids

Protective coatings for aluminium and as an anti-blocking agent

Wetting agent for leaching copper ores and as a froth flotation agent

To promote ore wetting and quicker breaking of the protective oxide layer

### Pharmaceutical Industry

Improve the properties and penetration of antimicrobial agents

Improve the properties of biochemicals, biocides, algicides, bacteriocides, and bacteriostats Improve the strength, homogeneity, and reduce the permeability of encapsulated materials Emulsify fluorochemical blood substitutes

### Agriculture and Forestry

Wetting agent for herbicides, fungicides, weed killers, hormone growth regulators, parasiticides, insecticides, germicides, bactericides, nematocides, microbiocides, defolients and fertilizers

As an ingredient in chemosterilents, insect repellents and toxicants

For wettable powder pesticides and chemical powders

Corrosion inhibitor for chemical applicators

Wetting agent for foliage

Wetting additive for live stock dips, or to wet sheep skins during desalination

Wetting adjuvant for manufacture of poywood veneer

Penetrant for preservative impregnation

Pulping aid

For cleaning tubes in paper making dyeing

Grease/oil repellents for paper

### Fire fighting

Wetting agent for fighting forest fires

Ingredient of AFFF (aqueous film forming foams) extinguishing agents

Component of fluoroprotein foams

Additives to dry chemical extinguishing agents

Agent in aerosol-type extinguishers

Wetting agent for sprinkler water

### Automotive, building maintenance and cleaning

Wetting agent for cleaning compositions

Additive for alkaline cleaners

Glass cleaner

Wetting agent for automobile waxes

Adjuvant to improve oil/water repellency of way

Lubricant/corrosion inhibitor for antifreeze

Rinse-aid for car washes

In dry cleaning compositions and solvent cleaners, for water displacement and foaming. May improve soil suspension and decrease redeposition

Foaming agents for pipe cleaning

Anti-mist film foamer for glass and plastics

In foams for dust suppresion

Cleaner for building exteriors

For acidic concrete cleaners

Air entrainment additive for low density concrete

Bubble foamer for air tracing, in ventilating systems

### Household, Cosmetic and personal products

Rinse-aid for dishwashing

Liquid polishing compositions

Floor polish leveling agent

Additive for alkaline oven cleaners

Synergistic improver for disinfectants

Carpet cleaners

Synergistic wetting agent in detergent formulations

Additive for protective coatings on metals (tarnish resistance, grease resistance)

Gloss and antistatic improver

Hair shampoo ingredient

Shaving foam ingredient

Oil and water repellent cosmetic powders ingredient

Ingredient for lotions or creams for skin or hair

Ingredient of skin protection creams

### Photography and graphic arts

Printing ink additive for ink flow and leveling, both aqueous and solvent based

Wetting agent for writing inks

To combat pigment flooding and flotation in printing inks

To form ink repellent surfaces for waterless lithoplates, or electrographic coatings

Prevent reticulation of gelatin layers and improve uniformity

Assist in film drying

Improve film coatings and reduce "Contraction flecks"

Wetting, leveling, anti-cratering assist agent

Surfactant for developer solutions

Photoemulsion stabilizer

Prevent photo-lubricant agglomeration

Coating aid in the preparation of multiple layer film elements

Antistatic wetting agent for film coatings

Antifogging agent for films

Bonding agent for fillers and fluoropolymer films

In coatings for nematic liquid crystal cells

The following examples are presented to illustrate the present invention. Unless otherwise indicated parts and precentages are by weight.

### Example 1

Into a reaction flask 57.8 g of 51.3% aqueous solution of 3-chloro-2-hydroxy-propyf.trimethyl ammonium chloride, 127.8 g water, 69.8 g of 1,1,2,2-tetrahydroperfluoroalkyl mercaptan (i.e. R_{f3}CH₂CH₂SH), and 31.2 g hexylene glycol are charged. The reaction mixture was maintained under a nitrogen atmosphere throughout the process. The above materials were stirred throughout the process. The above materials were stirred together at room temperature. With continued stirring, 25.7.g of 25% aqueous sodium hydroxide solution was slowly added into the mixture over a 15 minute period. The reaction mixture exothermed to 35°C and become homogeneous shortly after the addition was complete. The mixture was then stirred for an additional 2 hours at 45-50°C. A qualitative iodine test for unreacted mercaptan was negative indication complete conversion. The reaction product was cooled to room temperature and the pH was adjusted from 8.3 to 7.0 with glacial acetic acid. The resulting product was a clear, colorless, homogeneous solution of the following compositions.

30% of a compound of the formula 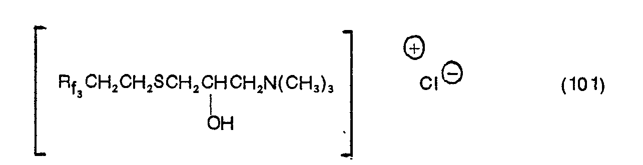 wherein R_{f3} is a mixture of
2 % perfluorobutyl
33% perfluorohexyl
37% perfluorohexyl
22% perfluorodecyl
5% perfluorododecyl and
1 % perfluorotetradecyl,
3% sodium chloride
10% hexylene glycol and
57% water.

The product was completely water miscible and very surface active, as indicated by the following measured data:

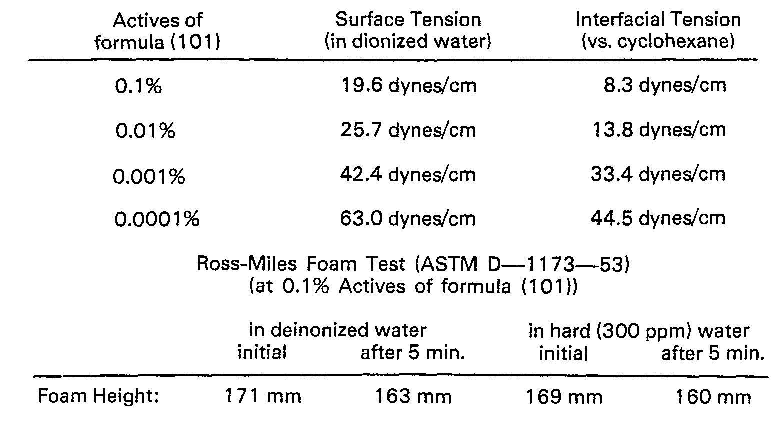

Analysis: 14.75% F calculated in product solution - 15.03% F average found

### Example 2

Into the reaction flask, the following items were charged: 36.2 g of a 52% aqueous solution of 3-chloro-2-hydroxy-propyl trimethyl ammonium chloride, 89.2 g water, 48.2 g of C₈F₁₇CH₂CH₂SH, and 21.1 g acetone. The reaction mixture was placed under a nitrogen atmosphere. As the above items_{'} were stirred together at room temperature, 16.0 g of 25% aqueous sodium hydroxide solution was slowly added over 15 minutes into the reaction mixture. The initially 2-layered system rapidly becomes homogeneous as the exothermic reaction reached 35°C. At this point, 1 ml hexylene glycol was added to reduce the viscosity to facilitate stirring. The mixture was then stirred for 2 hours at 50°C. The resulting product was transferred to a shallow dish and dried to a solid in a 60°C draft-oven followed by placement in a 70°C vacuum oven at 0.2 mm Hg.

The product, a white powder contains the compound of the formula 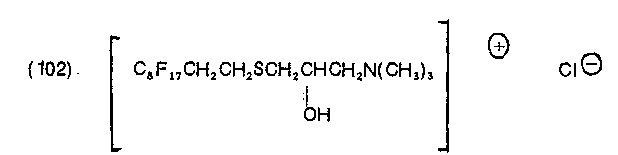 and sodium chloride by-product.

22 g of the dried solid was stirred in 200 ml refluxing dry chloroform to extract the pure cationic surfactant from the mixture which contained the by-product NaCI. The resulting chloroform solution was evaporated to solids in a rotary evaporator under reduced pressure. 0.9 g of pale-yellow solids were collected which was water-soluble and produced foaming.

The obtained mixture of the compound of formula (102) and sodium chloride produced an NMR spectrum generally consistent for the structure and had the following elemental analysis:

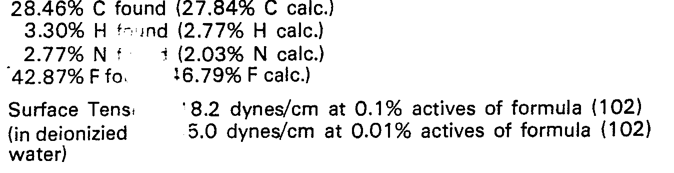

The chloroform extracted material (the fluorinated cationic compound only) also produced an NMR spectrum generally consistent for the structure and had the following elemental analysis:

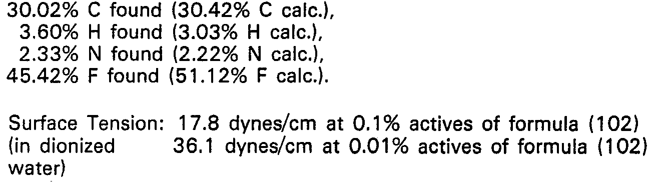

### Examples 3-9

The following series of examples demonstrates the effects of varying the solids content, solvent (hexylene glycol) content and water content on the appearance of the final product. These examples also demonstrate the trial experiments required to obtain a clear, homogeneous product. Each of these reactions was run similarly to the process described in Example 1. These examples are shown in Table I.

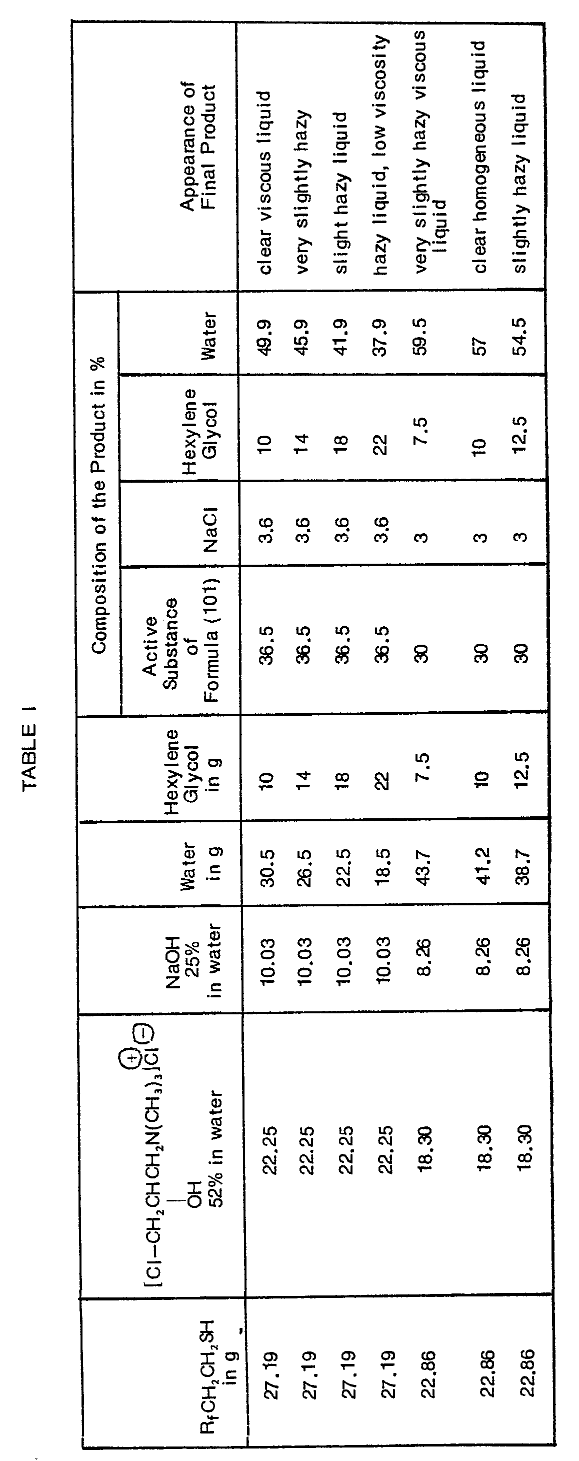

Examples 10-12

This series of examples demonstrates that bases other than sodium hydroxide can be used.

These Examples were also run according to the procedure described in Example 1. In each of these examples 18.56 g R_{f}CH₂CH₂SH, 15.19 g of 3-chloro-2-hydroxy-propyl trimethyl ammonium chloride at 52% in water, and 0.0428 equivalent of the various bases (in Table II) were used.

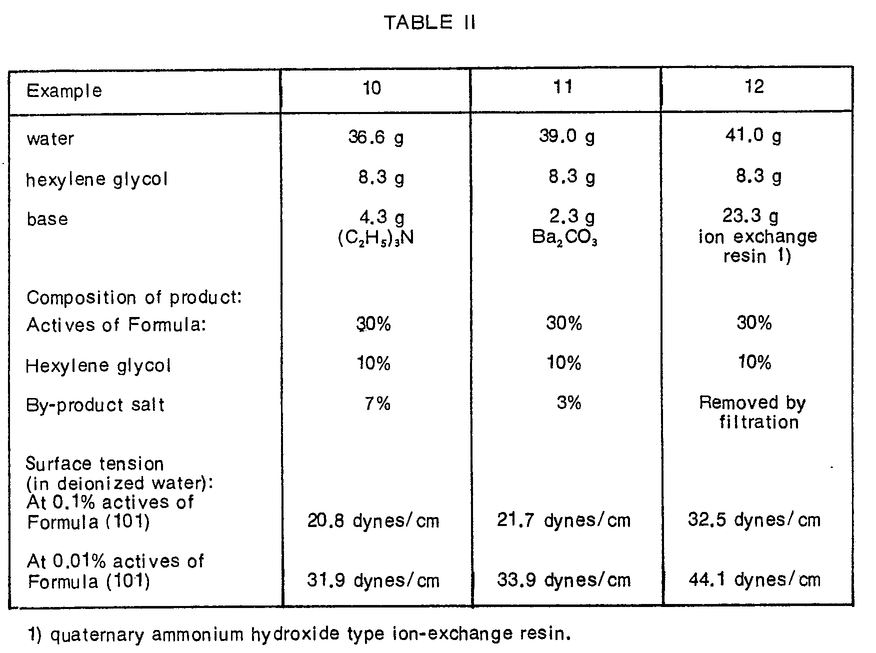

### Examples 13-15

This series of experiments demonstrates that a variety of water miscible solvents can be used as the co-solvent in these sytems.

These examples were also run according to the procedure described in Example 1.

Each of these examples contained:
18.56 g R_{f}CH₂CH₂SH
15.19 g [Cl―CH₂CH(OH)CH₂⊕N(CH₃)₃]Cl^{Θ} (52% in water)
6.85 g 25% aqueous sodium hydroxide solution
8.31 g of the various solvents (in Table III)

Composition of each:
30% actives of formula (101)
3% by-product sodium chloride
10% solvent (see Table III)
57% water

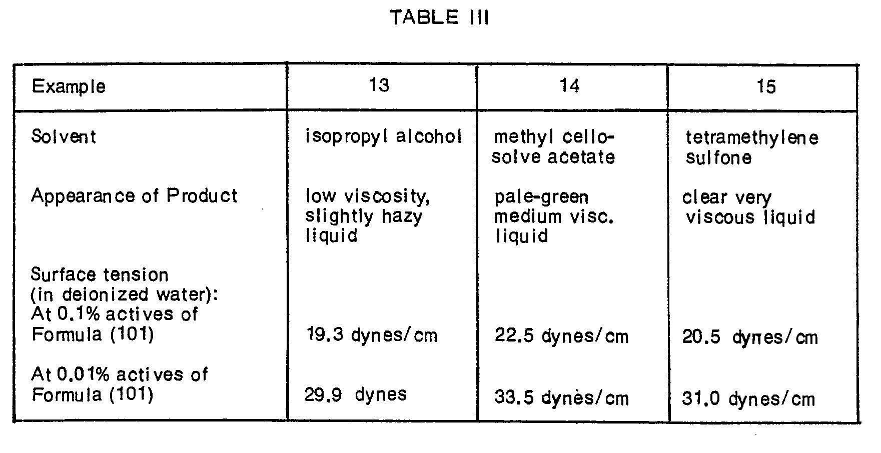

### Example 16

Into a glass bottle

17.11 g of a 52% aqueous solution of 3-chloro-2-hydroxy-propyl trimethyl ammonium chloride 24.64 g water

21.67 g of C₈F₁₇CH₂CH₂SH and

7.9 g of hexylene glycol were added. While stirring 7.71 g of 25% aqueous sodium hydroxide solution was added. The initially two-layered mixture rapidly became homogeneous and reached 45°C. The bottle was then placed in a 60°C shakerbath for 1 hour. The reulting clear. homogeneous solution showed a negative qualitative iodine test for unreacted mercaptan, indicating complete conversion.

### Composition of final product

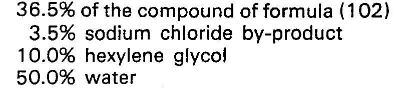

### Surface tension (in dionized water):

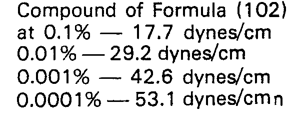

### Example 17

Into a flask, equipped with a magnetic stirrer, thermometer and nitrogen inlet 22.01 g of a 51.2% aqueous solution of 3-chloro-2-hydroxy-propyl trimethyl ammonium chloride, 38.63 g dionized water, 22.7 g of (CF₃)₂CFOCF₂CF₂CH₂CH₂SH and 10.11 isopropyl alcohol were charged, while stirring at room temperature, 9.60 g of 25% aqueous sodium hydroxide solution was added in over 5 minutes. The initially two-layered mixture rapidly became homogeneous and heated up to 24°C. The mixture was stirred for an additional 1 hour at 40°C. The resulting clear homogeneous solution showed a negative qualitative test (with iodine) for unreacted mecaptan indicating complete conversion.

A portion of the above product solution was dried to solids (50°C at 0.5 mm) for NMR analysis (solids contained both the active product of the formula 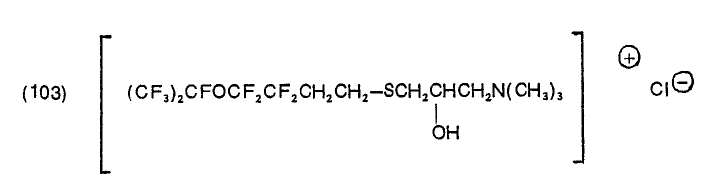 and NaCl by-product). The NMR spectrum was generally consistent for the above structure.

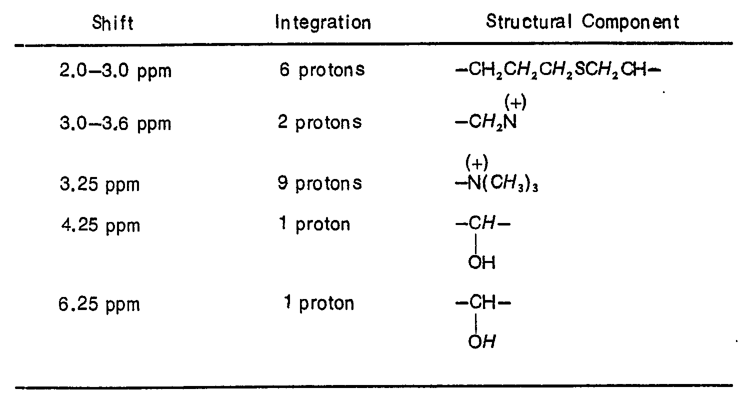

The remaining solution was pH adjusted from 11.2 to 6.9 with acetic acid.

Composition of Product solution:

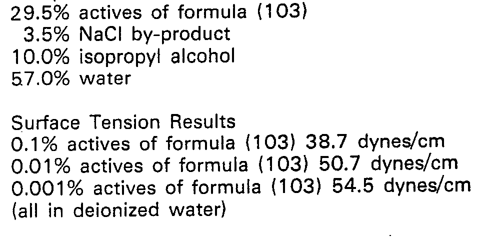

### Examples 18 to 24

Following the procedure of Example 2, the appropriate perfluoroalkyl thiols are reacted with the appropriate 3-chloro-2-hydroxy-propyl ammonium chlorides to yield products of the formula 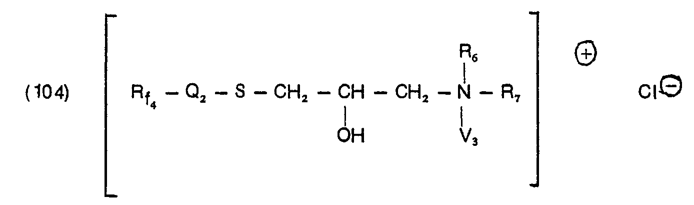 which are shown in the following Table IV.

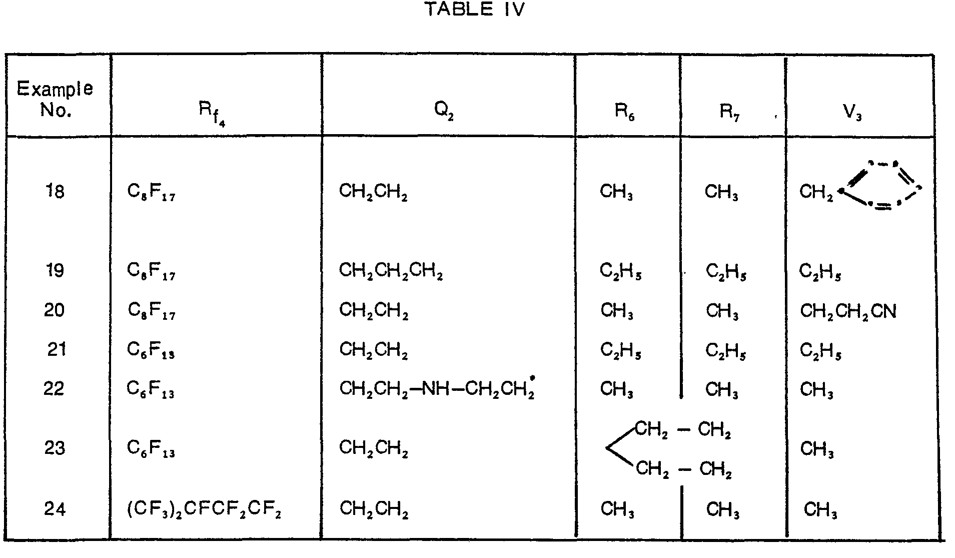

## Claims

1. A process for the manufacture of fluorinated cationic compounds of the formula 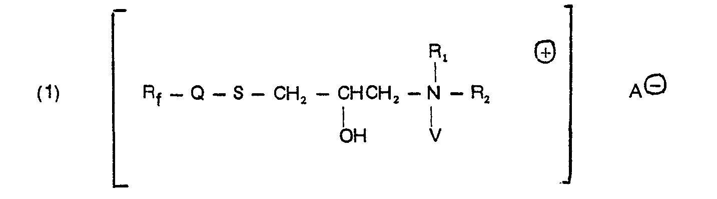 wherein R, is perfluoroalkyl of 1 to 18 carbon atoms or said perfluoroalkyl substituted by perfluoroalkoxy of 2 to 6 carbon atoms, Q is alkylene of 1 to 12 carbon atoms, alkylene thioalkylene of 2 to 12 carbon atoms, alkylene oxyalkylene of 2 to 12 carbon atoms, or alkylene iminoalkylene of 2 to 12 carbon atoms, wherein the imino group is optionally substituted by alkyl of 1 to 6 carbon atoms, V, R, and R₂ independently of one another represent alkyl of 1 to 20 carbon atoms which is optionally substituted by hydroxyl, cyano, halogen, or by alkoxy of 1 to 4 carbon atoms, or cycloalkyl, benzyl, phenyl or polyalkyleneoxy of 2 to 20 alkoxy units and alkylene units of 2 to 4 carbon atoms, or R, and R₂ together with the nitrogen atom to which they are attached, represent a 5- or 6-membered heterocyclic radical or R₁, R₂ and V, together with the nitrogen atom which links them, represent a pyridine ring and AID represents the anion of an organic or inorganic acid, which comprises reacting a mercaptan of the formula (2) R_{f}―Q―SH with a compound of the formula 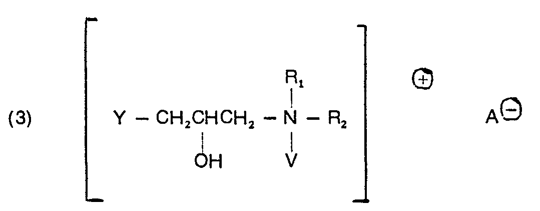 wherein R_{f} Q, R₁, R₂, V and A⁸ have the meanings indicated above and Y represents halogen.

2. A process according to claim 1, wherein R_{f} represents perfluoroalkyl group or a mixture of perfluoroalkyl groups of 3 to 18 carbon atoms.

3. A process according to claim 1, wherein Q represents alkylene or alkyl substituted alkylene of 2 to 8 carbon atoms.

4. A process according to claim 3, wherein Q is C₂―C₃-alkylene.

5. A process according to claim 1, wherein R₁, R₂ and V are independently of one another alkyl of 1 to 4 carbon atoms.

6. A process according to claim 1 for the manufacture of a fluorinated cationic compound of the formula 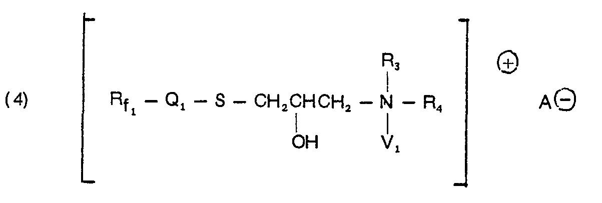 wherein R_{f}, is perfluoroalkyl group of 1 to 18 carbon atoms, Q₁ is alkylene or 2 or 3 carbon atoms, each of R₃ and R₄ independently represents alkyl of 1 to 4 carbon atoms, and Vᵢ is alkyl of 1 to 4 carbon atoms or benzyl and A⁸ is the anion of an organic or inorganic acid.

7. A process according to claim 6 for the manufacture of a fluorinated cationic compound of the formula 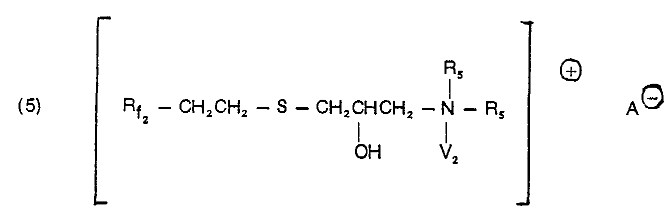 wherein R_{f2} is a perfluoroalkyl group of 4 to 14 carbon atoms and each of V₂ and R₅ is methyl or ethyl and A^{Θ} has the meaning given in claim 6.

8. A process according to claim 7, wherein R_{f2} is perfluorobutyl, perfluorohexyl, perfluorooctyl, perfluorodecyl, perfluorododecyl, perfluorotetradecyl or a mixture of these substituents, V₂ and R₅ represents methyl or ethyl and A^{e} represents a chloride ion.

9. Use of the fluorinated cationic compounds prepared according to the process of any one of claims 1 to 8 as surfactants.

## Revendications

1. Procédé pour la fabrication de composés cationiques fluorés de formule: 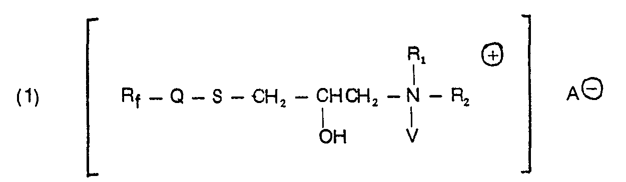 dans laquelle R_{f} est un groupe perfluoroalkyle ayant 1 à 18 atomes de carbone, ou ce groupe pertluo- roalkyle substitué par un groupement perfluoroalcoxy ayant 2 à 6 atomes de carbone, Q est un groupe alkylène ayant 1 à 12 atomes de carbone, alkylènethioalkylène ayant 2 à 12 atomes de carbone, alkylène-oxyalkylène ayant 2 à 12 atomes de carbone, ou alkylène-iminoalkylène ayant 2 à 12 atomes de carbone, où le groupe imino est éventuellement substitué par un radical alkyle ayant 1 à 6 atomes de carbone, V, R₁ et R₂ indépendamment l'un de l'autre représentent des groupes alkyle ayant 1 à 20 atomes de carbone qui sont éventuellement substitués par des radicaux hydroxyle, cyano, halogéno, ou par des radicaux alcoxy ayant 1 à 4 atomes de carbone, ou des groupes cycloalkyle, benzyle, phényle ou polyalkylèneoxy ayant 2 à 20 unités alcoxy et des unités alkylène ayant 2 à 4 atomes de carbone, ou bien R₁ et R₂ ensemble avec l'atome d'azote auquel ils sont reliés, représentent un radical hétérocyclique à 5 ou 6 chaînons, ou bien R₁, R₂ et V, ensemble avec l'atome d'azote qui les relie représentent un noyau pyridine, et A⁸ représente l'anion d'un acide organique ou minéral, procédé qui comprend la réaction d'un mercaptan ayant la formule: (2) R_{f}―Q―SH avec un composé de formule: 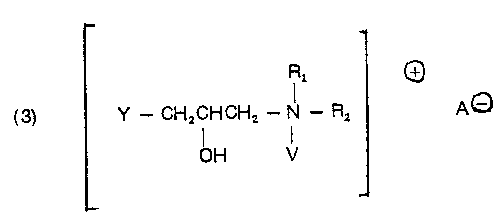 dans lesquels R_{f}, Q, R₁, R₂, V et A^{G} ont les significations indiquées ci-dessus, et Y représente un halogène.

2. Procédé selon la revendication 1, dans lequel R_{f} représente le groupe perfluoro-alkyle ou un mélange de groupes perfluoroalkyle ayant 3 à 18 atomes de carbone.

3. Procédé selon la revendication 1, dans lequel Q représente un groupe alkylène ou un groupe alkylène ayant 2 à 8 atomes de carbone substitué par un radical alkyle.

4. Procédé selon la revendication 3, dans lequel Q est le groupe C₂―C₃-alkylène.

5. Procédé selon la revendication 1, dans lequel R₁, R₂ et V sont indépendamment l'un de l'autre des groupes alkyle ayant 1 à 4 atomes de carbone.

6. Procédé selon la revendication 1 pour la fabrication d'un composé cationique fluoré ayant la formule: 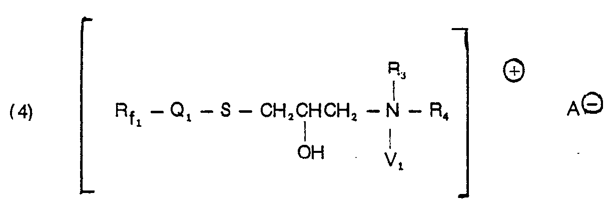 dans laquelle R_{f}, représente un groupe per^{f}luoroalkyle ayant 1 à 18 atomes de carbone, Q₁ est un groupe alkylène ayant 2 à 3 atomes de carbone, chaque symbole R₃ et R₄ représente indépendamment un groupe alkyle ayant 1 à 4 atomes de carbone, et V₁ est un groupe alkyle ayant 1 à 4 atomes de carbone ou un groupe benzyle, et A⁸ est l'anion d'un acide .organique ou minéral.

7. Procédé selon la revendication 6 pour la fabrication d'un composé cationique fluoré de formule: 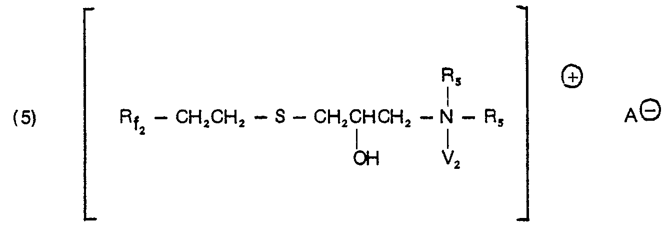 dans laquelle R_{f2} représente un groupe perfluoroalkyle ayant 4 à 14 atomes de carbone, et chaque symbole V₂ et R₅ est un groupe méthyle ou éthyle, et A⁸ a la signification donnée dans la revendication 6.

8. Procédé selon la revendication 7, dans lequel R_{f2} est un groupe perfluorobutyle, perfluoro- hexyle, perfluorooctyle, perfluorodécyle, perfluorododécyle, perfluorotétradécyle ou un mélange de ces substituants, V₂ et R₅ représentent des groupes méthyle ou éthyle, et A⁸ représente un ion chlorure.

9. Utilisation des composés cationiques fluorés préparés selon le procédé de l'une quelconque des revendications 1 à 8, comme tensio-actifs.

## Patentansprüche

1. Verfahren zur Herstellung von fluorierten kationischen Verbindungen der Formel 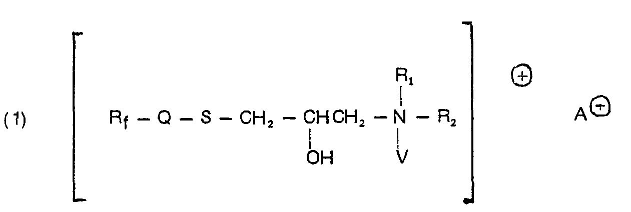 worin R₁ Perfluoralkyl mit 1 bis 18 Kohlenstoffatomen, gegebenenfalls substituiert mit Perfluoralkoxy mit 2 bis 6 Kohlenstoffatomen und Q Alkylen mit 1 bis 12 Kohlenstoffatomen, Alkylenthioalkylen mit 2 bis 12 Kohlenstoffatomen, Alkylenoxyalkylen mit 2 bis 12 Kohlenstoffatomen oder Alkyleniminoalkylen mit 2 bis 12 Kohlenstoffatomen ist, worin die Iminogruppe gegebenenfalls mit Alkyl mit 1 bis 6 Kohlenstoffatomen substituiert ist, V, Rₜ und R₂ unabhängig voneinander Alkyl mit 1 bis 20 Kohlenstoffatomen, das gegebenenfalls mit Hydroxyl, Cyan, Halogen oder Alkoxy mit 1 bis 4 Kohlenstoffatomen substituiert ist, oder Cycloalkyl, Benzyl, Phenyl oder Polyalkylenoxy mit 2 bis 20 Alkylenoxyeinheiten mit 2 bis 4 Kohlenstoffatomen sind, oder R₁ und R₂ zusammen mit dem Stickstoffatom, mit dem sie verbunden sind, einen 5- oder 6-gliedrigen heterocyclischen Rest bedeuten, oder R,, R und V zusammen mit dem Stickstoffatom, mit dem sie verbunden sind, einen Pyridinring bedeuten, und A⁸ das Anion einer anorganischen oder organischen Säure ist, dadurch gekennzeichnet, dass man ein Merkaptan der Formel (2) R_{f}―Q―SH mit einer Verbindung der Formel 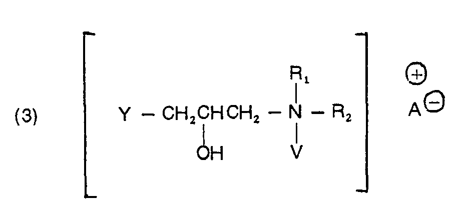 umsetzt, worin R_{f}, Q, R₁, R₂, V und A^{Θ} die angegebenen Bedeutungen haben und Y Halogen bedeutet.

2. Verfahren nach Anspruch 1, dadurch gekennzeichnet, dass R, eine Perfluoralkylgruppe oder ein Gemisch von Perfluoralkylgruppen mit 3 bis 18 Kohlenstoffatomen ist.

3. Verfahren nach Anspruch 1, dadurch gekennzeichnet, dass Q Alkylen oder alkylsubstituiertes Alkylen mit 2 bis 8 Kohlenstoffatomen ist.

4. Verfahren nach Anspruch 3, dadurch gekennzeichnet, dass Q Alkylen mit 2 oder 3 Kohlenstoffatomen ist.

5. Verfahren nach Anspruch 1, dadurch gekennzeichnet, dass R₁, R₂ und V unabhängig voneinander Alkyl mit 1 bis 4 Kohlenstoffatomen sind.

6. Verfahren nach Anspruch 1 zur Herstellung von fluorierten kationischen Verbindungen der Formel 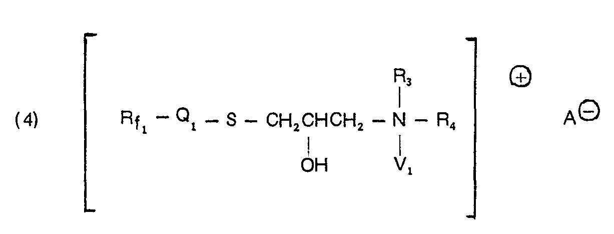 worin R_{ft} Perfluoralkyl mit 1 bis 18 Kohlenstoffatomen und Q₁ Alkylen mit 2 bis 3 Kohlenstoffatomen ist, R₃ und R₄ unabhängig voneinander Alkyl mit 1 bis 4 Kohlenstoffatomen sind, V₁ Alkyl mit 1 bis 4 Kohlenstoffatomen oder Benzyl und A⁸ das Anion einer anorganischen oder organischen Säure ist.

7. Verfahren nach Anspruch 6 zur Herstellung von fluorierten kationischen Verbindungen der Formel 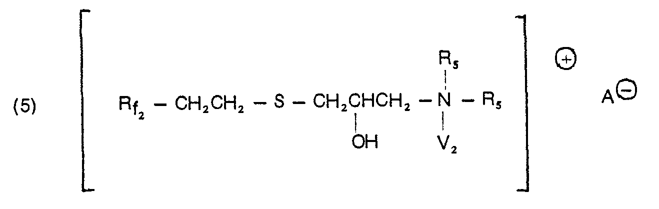 worin R_{f2} Perfluoralkyl mit 4 bis 14 Kohlenstoffatomen ist, V₂ und R₅ Methyl oder Aethyl sind und A die in Anspruch 6 angegebene Bedeutung hat.

8. Verfahren nach Anspruch 7, dadurch gekennzeichnet, dass R_{f2} Perfluorbutyl, Perfluorhexyl, Perfluoroctyl, Perfluordecyl, Perfluordodecyl, Perfluortetradecyl oder ein Gemisch dieser Reste ist, V₂ und R₅ Methyl oder Aethyl sind und A⁸ das Chloridanion ist.

9. Verwendung der nach den Verfahren der Ansprüche 1 bis 8 hergestellten fluorierten kationischen Verbindungen als oberflächenaktive Hilfsmittel.
